# EUROPÄISCHE PATENTSCHRIFT

(11) **EP 1 475 053 B1**
(45) Veröffentlichungstag und Bekanntmachung des Hinweises auf die Patenterteilung: **25.03.2009**
(21) Anmeldenummer: 04010068.7
(22) Anmeldetag: 28.04.2004
(51) Int. Cl.: A61F 2/04, A61F 2/06, B23K 26/00

(54) **Verfahren zur Herstellung eines medizinischen Implantates**
Method of manufacturing a medical implant
Procédé de fabrication d'un implant médical

(30) Priorität: 07.05.2003 DE 10320262
(43) Veröffentlichungstag der Anmeldung: 10.11.2004
(73) Patentinhaber: MeKo Laserstrahl-Materialbearbeitungen, 31157 Sarstedt (DE)
(72) Erfinder: Meyer-Kobbe, Clemens, Dr., 31157 Sarstedt (DE)
(74) Vertreter: Scheffler, Jörg

(56) Entgegenhaltungen:
- EP-A- 0 647 439
- EP-A- 0 850 604
- WO-A-02/43619
- US-A- 6 099 561

## Beschreibung

Die Erfindung betrifft ein Verfahren zur Herstellung eines medizinischen Implantates aus Metall oder Kunststoff, beispielsweise ein Stent, eine Hüftprothese oder ein Knochennagel, wobei zunächst die inneren und/oder äußeren Oberflächen des Implantates poliert werden und anschließend in das Implantat mittels eines elektromagnetischen Energieeintrages gezielt in diesen Bereichen der inneren und/oder äußeren Oberflächen des Implantates eine gewünschte Rauheit und/oder Strukturierung eingebracht und anschließend eine Beschichtung in den Bereichen aufgebracht wird.

Jährlich erleiden in Deutschland mehrere hunderttausend Menschen einen Herzinfarkt. Es handelt sich hierbei um eine schwere Durchblutungsstörung des Herzens, die zumeist durch Verengung oder sogar Verschluss eines Herzkranzgefäßes entsteht. In Fachkreisen spricht man hier von Arterienverkalkung oder Arteriosklerose.

Gesunde Arterien sind flexibel. Durch falsche Ernährung, mangelnde Bewegung und nicht zuletzt auch durch den natürlichen Alterungsprozess sammeln sich Fettsäuren, Cholesterin und Kalziumsalze in den Blutgefäßen an. In Folge dessen verlieren die Arterien an Flexibilität, die Gefäßwände verdicken und verengen sich. Der Blutfluss wird zunehmend behindert und Organe werden nur mangelhaft mit Blut versorgt. An den verengten Gefäßwänden kann es außerdem zu Gerinnungsprozessen kommen. Durch die folgliche Entstehung von Blutgerinnseln können die Gefäße sogar vollständig verschlossen werden, deshalb stirbt Herzmuskelgewebe ab und es kommt zum Herzinfarkt.

Um verschlossene Gefäße wieder zu öffnen, werden Katheder eingesetzt, an deren Spitze sich ein Ballon befindet. Dehnt man diesen Ballon aus, wird die Gefäßverengung gesprengt. Damit das Blutgefäß nach dem Öffnen nicht in sich zusammen fällt, setzt man so genannte "Stents" als Gefäßstütze ein. In der Praxis weisen diese Stents beispielsweise ein feines Drahtgeflecht aus einem medizinischen Edelstahl auf, damit die Stützfunktion gewährleistet ist.

Zur besseren Körperverträglichkeit weisen die Stents eine polierte Oberfläche und verrundete Ecken und Kanten auf, um den Blutfluss so wenig wie möglich zu beeinträchtigen. Die Oberflächenbearbeitung erfolgt beispielsweise mechanisch oder elektrochemisch, wobei die Oberflächeneigenschaften der äußeren wie auch der inneren Oberfläche im Wesentlichen übereinstimmen.

Das verwendete Material kann aus Edelstahl, Nickel-Titan-Legierungen, Formgedächtnislegierungen oder anderen Legierungen bestehen. Die typischen Rohrdurchmesser betragen zwischen 1 und 10 mm bei einer Wandstärke von ca. 100 µm. Mit Stent-Lasersystemen können gratfreie Schnitte unterhalb 20 µm Breite erzielt werden. Schnittgeschwindigkeiten von mehreren mm/sec sind möglich.

Es sind insbesondere auch bereits Gefäßstützen mit unterschiedlichen passiven Beschichtungen bekannt. Beispielsweise sind Versuche unternommen worden, die Gefäßstütze mit Gold zu beschichten, um so eine geringere Restenose-Rate zu erreichen. Weiterhin sind zu diesem Zweck auch Stents bekannt, die mit Keramiken oder polykristallinen Diamantschichten beschichtet sind.

Darüber hinaus wird mit radioaktiven Stents experimentiert, um so durch lokale Strahlentherapie - über einen radioaktiven Drahtanteil oder indem man Radioaktivität direkt über einen Stent platziert - die Restenose-Rate zu vermindern.

Weiterhin sind auch Stents bekannt, deren Oberflächen mit Medikamenten, beispielsweise Phosphorylcholin oder mit Heparin, beschichtet sind. Nach der Stentimplantation werden diese Medikamente freigesetzt, um eine erneute Verengung der Gefäße zu verhindern. Außerdem ist es auch bekannt, Stents mit einer antiproliferativen Substanz zu beschichten und somit die Möglichkeit zu schaffen, die Proliferation der glatten Muskelzelle einzuschränken.

Zur Vorbereitung der Beschichtung ist es erforderlich, die Oberfläche hinsichtlich der gewünschten, auf die Beschichtung abgestimmten Adhäsionseigenschaften vorzubereiten. Dabei erweist es sich jedoch als hinderlich, dass einerseits durch elektrochemische Verfahren eine oberflächenselektive Bearbeitung nicht möglich ist. Andererseits erweisen sich mechanische Verfahren zur Oberflächenbearbeitung in der Praxis als problematisch, weil durch den Abtrag eine Kontamination eingebracht wird.

Ein gattungsgemäßes Verfahren zur Herstellung eines medizinischen Implantates, beispielsweise eines Stents, beschreibt die EP 0 850 604 A2, bei dem zunächst die Oberflächen des Implantates poliert werden und anschließend in das Implantat mittels eines elektromagnetischen Energieeintrages gezielt in diesen Bereichen der inneren und/oder äußeren Oberflächen des Implantates eine gewünschte Rauheit und/oder Strukturierung eingebracht und anschließend eine Beschichtung in den Bereichen aufgebracht wird. Zum Einbringen der Rauheit oder Strukturierung wird ein Laser eingesetzt.

Weiterhin beschreibt auch die DE 202 00 220 U1 ein solches als Stent ausgeführtes Implantat, welches mit einer Verstrebungen aufweisenden Wandung, wobei in der Wandung oder in den Verstrebungen Bereich zur Aufnahme einer oder mehrerer Medikamente vorgesehen sind, wobei diese Bereich die Wandung oder Verstrebung durchringen.

Durch die DE 197 45 294 A1 ist ein Verfahren zur Herstellung feinstrukturierter medizinischer Implantate durch Laser Materialbearbeitung, insbesondere Laserschneiden von bioresorbierbaren Gefäßstützen, bekannt. Dabei werden die Laserparameter derart eingestellt, dass eine schmelzfreie materialschonende Bearbeitung des Werkstoffes stattfindet.

Ferner beschreibt die WO 02/30328 A1 Möglichkeiten zur Oberflächenglättung bei Stents durch kurzzeitiges Laserumschmelzen einer dünnen Randschicht.

Der Erfindung liegt die Aufgabe zugrunde, ein Verfahren zur Herstellung eines medizinischen Implantates der eingangs genannten Art derart wesentlich zu verbessern. Insbesondere soll dadurch das Aufbringen einer Beschichtung vereinfacht werden.

Diese Aufgabe wird erfindungsgemäß mit einem Verfahren gemäß den Merkmalen des Anspruchs 1 gelöst. Die weitere Ausgestaltung der Erfindung ist den Unteransprüchen zu entnehmen.

Erfindungsgemäß wird bei dem Verfahren zur Herstellung eines medizinischen Implantates aus Metall oder Kunststoff, beispielsweise ein Stent, eine Hüftprothese oder ein Knochennagel, bei dem zunächst die inneren und/oder äußeren Oberflächen des Implantates poliert werden und anschließend in das Implantat mittels eines elektromagnetischen Energieeintrages gezielt in diesen Bereichen der inneren und/oder äußeren Oberflächen des Implantates eine gewünschte Rauheit und/oder Strukturierung eingebracht und anschließend eine Beschichtung in den Bereichen aufgebracht wird, die Rauheit und/oder Strukturierung mittels eines Kurzpulslasers eingebracht und eine flächenartige Strukturierung pro Laserpuls erzeugt, wobei durch die Form und Überlagerung mehrerer Laserpulse die gewünschte Rauheit und/oder Strukturierung erreicht wird.

Dabei wird einfacher Weise die gewünschte Rauheit oder Strukturierung gezielt in solchen Bereichen realisiert, die für das Aufbringen einer Beschichtung vorgesehen sind. Hierzu werden zunächst die inneren sowie äußeren Oberflächen des Implantates poliert, so dass der Blutfluss so wenig wie möglich beeinträchtigt wird. Zugleich ermöglicht die gezielte Rauheit und Strukturierung das Aufbringen der Beschichtung, wobei insbesondere eine mechanische Bearbeitung, insbesondere ein mechanisches Abtragen oberflächenaher Schichten entfällt. Die mittels des elektromagnetischen Energieeintrages eingebrachte Rauheit oder Strukturierung, die insbesondere weniger als 5 µm beträgt, erfolgt dabei ohne Rückstände durch Verdampfung, so dass eine Kontaminierung des Implantates weitgehend ausgeschlossen werden kann. Grundsätzlich kann die so geschaffene Rauheit oder Strukturierung durch ihre das Einwachsen des Stents begünstigende Rauheit der äußeren Oberfläche auch ohne eine Beschichtung eingesetzt werden. Die Pulsdauer und Pulsenergie des elektromagnetischen Energieeintrages ist derart kurz und auf einem hohen Energieniveau, dass die Oberfläche schmilzt und verdampft. Durch einen hohen Verdampfungsanteil bei dem elektromagnetischen Energieeintrag wird die gewünschte Rauheit oder Strukturierung erzeugt. Durch Einsetzen eines Stiftes in das Implantat kann dabei eine Beschädigung der inneren Oberfläche des Implantates durch den elektromagnetischen Energieeintrag vermieden werden.

Hierbei erweist es sich als besonders Erfolg versprechend, dass die Rauheit oder Strukturierung mittels eines Festkörperlaser, Excimerlaser oder Femtosekundenlaser eingebracht wird. Zum Einsatz kommen unter anderem gütegeschaltete Festkörperlaser, beispielsweise Beschriftungssystem mit Nd:YAG-Laser, und Excimerlaser oder andere Kurzpulslaser, beispielsweise Femtosekundenlaser. Die Verfahrensparameter werden dabei insbesondere derart eingestellt, dass die gewünschte Rauheit oder Strukturierung mit einem geringen Materialabtrag erreicht wird, wobei das abgetragene Material insbesondere verdampft wird, so dass keine unerwünschten Rückstände auftreten. Insbesondere beim Einsatz des Excimerlasers können durch hohe Pulsenergien große Flächen pro Laserpuls bearbeitet werden, wobei die Strukturierung der Oberfläche durch die Form und Überlagerung der Laserpulse wesentlich bestimmt werden. Die Verfahrensparameter sind vorzugsweise so zu wählen, dass bei der Oberflächenstrukturierung möglichst wenig Material durch Verdampfung abgetragen wird.

Dabei erweist es sich als besonders zweckmäßig, wenn die Rauheit oder Strukturierung durch einzelne Ausnehmungen mit einer vorbestimmten Beschaffenheit und Verteilung eingebracht werden. Hierdurch können die Eigenschaften des Implantates an die individuellen Anforderungen optimal angepasst werden. Beispielsweise kann das Einwachsen des Implantates durch die Strukturierung in bestimmten Bereichen und damit der medizinische Heilungsfortschritt begünstigt werden.

Dabei wird eine besonders günstige Weiterbildung der Erfindung auch dadurch realisiert, dass die Ausnehmung einen Saum mit einer an die Beschichtung angepassten Rauheit aufweist. Hierdurch kann die Beschichtung in einfacher Weise durch den an diesen angepassten Saum fixiert werden, so dass der Aufwand für die Beschichtung wesentlich reduziert werden kann.

Eine besonders praxisgerechte Ausgestaltung der vorliegenden Erfindung wird auch dadurch erreicht, dass die Strukturierung rasterförmig, linienförmig oder stochastisch eingebracht wird. Hierdurch wird eine für die Fixierung optimal geeignete Strukturierung erzeugt, die an die jeweils gewünschte Verteilung bzw. Konzentration der Beschichtung in einfacher Weise angepasst werden kann. Der Heilungsverlauf wird dadurch wesentlich verbessert. Dabei bestimmen der Abstand der einzelnen Laserpulse und das Scanraster zusammen mit den gewählten Laserparametern die Oberflächenstruktur.

In ebensolcher Weise wird eine besonders gut geeignete Ausführungsform dadurch erreicht, dass die Rauheit oder Strukturierung selektiv in einzelne Bereiche eingebracht wird, um so die Beschichtung lediglich in solchen Bereichen des Implantates aufzubringen, die für die jeweilige Indikation erforderlich ist. Unnötige Beschichtungen werden dadurch vermieden.

Zugleich erweist es sich als besonders praxisgerecht, wenn mittels des elektromagnetischen Energieeintrages Kanten und Stege des Implantates verrundet werden. Hierdurch können in einem einzigen Arbeitschritt sowohl die gewünschte Rauheit und Strukturierung eingebracht als auch mögliche Grate insbesondere im Bereich der Durchbrechungen des Implantates mit dem gewünschten körperverträglichen Radius versehen werden. Der Herstellungsprozess kann dadurch weiter vereinfacht werden.

Dabei hat sich eine praktische Ausgestaltung als besonders sinnvoll erwiesen, bei der die Rauheit oder Strukturierung, insbesondere einzelne Ausnehmungen entsprechend dem erwartungsgemäßen Heilungsfortschritt eingebracht werden. Hierdurch kann die Bearbeitung der Implantatoberfläche auf solche Bereiche beschränkt werden, die in zweckmäßiger Weise mit der Beschichtung versehen werden. Die Strukturierung kann dadurch weiter vereinfacht werden.

Dabei können in ebenfalls besonders praxisorientierter Form die Ausnehmungen entsprechend dem gewünschten Beschichtungsvolumen eingebracht werden, um so insbesondere die Verteilung der in der Beschichtung enthaltenen Wirkstoffe örtlich verteilt zu realisieren.

Dabei kann weiterhin auf das Implantat eine aktive oder passive Beschichtung aufgebracht werden, so dass diese neben einer gezielten Oberflächenbeschaffenheit zumindest bereichsweise auch geeignete Wirkstoffe freisetzt, die den Heilungsprozess verbessern.

Die Beschreibung der Oberflächenstrukturierungen ist nicht abschließend. Vielmehr kann die vorteilhafte Wirkung bei Stents auch auf andere Implantate wie beispielsweise Knochennägel, Hüftprothesen und Knochenplatten angewandt werden.

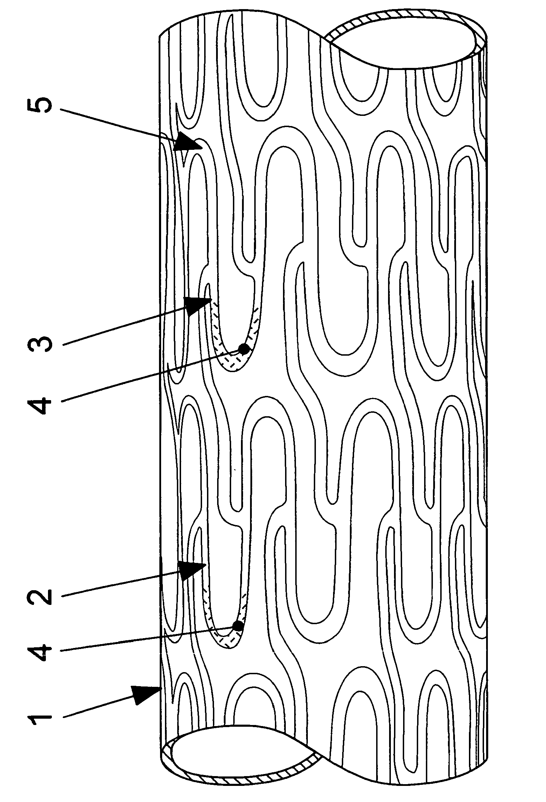

In der Zeichnung ist ein Ausführungsbeispiel der Erfindung dargestellt und wird im Folgenden näher beschrieben. Die Zeichnung zeigt in einer Draufsicht ein erfindungsgemäßes medizinisches Implantat 1, insbesondere eine Gefäßstütze aus Metall oder Kunststoff. Das Implantat 1 weist einzelne Bereiche 2, 3 mit einer Strukturierung 4 auf, durch die das problemlose Aufbringen einer nicht gezeigten Beschichtung ermöglicht wird. Hierzu wird zunächst mittels eines Laserstrahls gezielt in den Bereichen 2, 3 einer äußeren Oberfläche 5 des Implantates 1 die gewünschte Strukturierung 4 eingebracht und anschließend die Beschichtung in diesen Bereichen 2, 3 mittels an sich bekannter Verfahren aufgebracht.

## Patentansprüche

1. Verfahren zur Herstellung eines medizinischen Implantates (1) aus Metall oder Kunststoff, beispielsweise ein Stent, eine Hüftprothese oder ein Knochennagel, wobei zunächst die inneren und/oder äußeren Oberflächen (5) des Implantates poliert werden und anschließend in das Implantat mittels eines elektromagnetischen Energieeintrages gezielt in diesen Bereichen der inneren und/oder äußeren Oberflächen des Implantates eine gewünschte Rauheit und/oder Strukturierung (4) eingebracht und anschließend eine Beschichtung in den Bereichen aufgebracht wird, **dadurch gekennzeichnet, dass** die Rauheit und oder Strukturierung mittels eines Kurzpulslasers eingebracht und eine flächenartige Strukturierung pro Laserpuls erzeugt wird, wobei durch die Form und Überlagerung mehrerer Laserpulse die gewünschte Rauheit und / oder Strukturierung erreicht wird.

2. Verfahren nach Anspruch 1, **dadurch gekennzeichnet, dass** die Rauheit und / oder Strukturierung mittels eines Festkörperlaser, Excimerlaser oder Femtosekundenlaser eingebracht wird.

3. Verfahren nach den Ansprüchen 1 oder 2, **dadurch gekennzeichnet, dass** die Rauheit und / oder Strukturierung durch Ausnehmungen mit einer vorbestimmten Beschaffenheit und Abständen eingebracht werden.

4. Verfahren nach zumindest einem der vorhergehenden Ansprüche, **dadurch gekennzeichnet, dass** die Ausnehmung einen Saum mit einer an die Beschichtung angepassten Rauheit aufweist.

5. Verfahren nach zumindest einem der vorhergehenden Ansprüche, **dadurch gekennzeichnet, dass** die Strukturierung rasterförmig, linienförmig oder stochastisch eingebracht wird.

6. Verfahren nach zumindest einem der vorhergehenden Ansprüche, **dadurch gekennzeichnet, dass** die Rauheit und / oder Strukturierung selektiv in einzelne Bereiche eingebracht wird.

7. Verfahren nach zumindest einem der vorhergehenden Ansprüche, **dadurch gekennzeichnet, dass** mittels des elektromagnetischen Energieeintrages Kanten und Stege des Implantates verrundet werden.

8. Verfahren nach zumindest einem der vorhergehenden Ansprüche, **dadurch gekennzeichnet, dass** die Rauheit und / oder Strukturierung, insbesondere einzelne Ausnehmungen entsprechend dem erwartungsgemäßen Heilungsfortschritt eingebracht und die Bearbeitung der Implantatoberfläche auf solche Bereiche beschränkt werden, die in zweckmäßiger Weise mit der Beschichtung versehen werden.

9. Verfahren nach Anspruch 8, **dadurch gekennzeichnet, dass** die Ausnehmungen entsprechend dem gewünschten Beschichtungsvolumen eingebracht werden.

10. Verfahren nach zumindest einem der vorhergehenden Ansprüche, **dadurch gekennzeichnet, dass** eine aktive und / oder passive Beschichtung aufgebracht wird.

11. Verfahren nach zumindest einem der vorhergehenden Ansprüche, **dadurch gekennzeichnet, dass** die inneren und/oder äußeren Oberflächen des Implantates mechanisch und/oder elektrochemisch poliert werden.

12. Verfahren nach zumindest einem der vorhergehenden Ansprüche, **dadurch gekennzeichnet, dass** die gesamte Oberfläche des Implantates poliert wird.

13. Verfahren nach zumindest einem der vorhergehenden Ansprüche, **dadurch gekennzeichnet, dass** die Rauheit und/oder Strukturierung mittels des elektromagnetischen Energieeintrages mit einer Pulsdauer kleiner 1 µs eingebracht wird und 0,5 bis 5 µm beträgt.

## Claims

1. Method for manufacturing a medical implant (1) composed of metal or plastic, for example a stent, an artificial hip or a bone pin, wherein firstly the inner and/or outer surfaces (5) of the implant are polished and subsequently a desired degree of roughness and/or structure (4) is applied to the implant by means of an electromagnetic application of energy, selectively in these regions of the inner and/or outer surfaces of the implant, and subsequently coating is applied to the regions, **characterized in that** the roughness and/or structuring is applied by means of a short pulse laser, and surface-like structuring is generated per laser pulse, wherein the desired roughness and/or structuring is achieved by means of the shape and superimposition of a plurality of laser pulses.

2. Method according to Claim 1, **characterized in that** the roughness and/or structuring is applied by means of a solid-state laser, an excimer laser or fernto-second laser.

3. Method according to Claim 1 or 2, **characterized in that** the roughness and/or structuring is introduced through cutouts with a predetermined character and predetermined intervals.

4. Method according to at least one of the preceding claims, **characterized in that** the cutout has a border with a roughness which is adapted to the coating.

5. Method according to at least one of the preceding claims, **characterized in that** the structuring is applied in grid form, linearly or stochastically.

6. Method according to at least one of the preceding claims, **characterized in that** the roughness and/or structuring is applied selectively to individual regions.

7. Method according to at least one of the preceding claims, **characterized in that** edges and webs of the implant are rounded by means of the electromagnetic application of energy.

8. Method according to at least one of the preceding claims, **characterized in that** the roughness and/or structuring, in particular individual cutouts corresponding to the anticipated progress in healing, are applied and the processing of the implant surface is restricted to those regions which are expediently provided with the coating.

9. Method according to Claim 8, **characterized in that** the cutouts are applied in accordance with the desired coating volume.

10. Method according to at least one of the preceding claims, **characterized in that** an active and/or passive coating is applied.

11. Method according to at least one of the preceding claims, **characterized in that** the inner and/or outer surfaces of the implant are polished mechanically and/or electrochemically.

12. Method according to at least one of the preceding claims, **characterized in that** the entire surface of the implant is polished.

13. Method according to at least one of the preceding claims, **characterized in that** the roughness and/or structuring by means of the electromagnetic application of energy is applied with a pulse length of less than 1 µs and are 0.5 to 5 µm.

## Revendications

1. Procédé de fabrication d'un implant médical (1) en métal ou en matière synthétique, par exemple une prothèse endovasculaire, une prothèse de la hanche ou un clou médullaire, dans lequel la surface intérieure et/ou la surface extérieure (5) de l'implant sont d'abord polies pour former ensuite de manière contrôlée une rugosité et/ou une structuration (4) souhaitées dans l'implant au moyen d'un apport d'énergie électromagnétique dans ces parties de la surface intérieure et/ou de la surface extérieure et pour appliquer ensuite un revêtement dans ces parties,
**caractérisé en ce que**
la rugosité et/ou la structuration sont formées au moyen d'un laser à brèves impulsions, une structuration de surface est formée lors de chaque impulsion laser, la forme des impulsions laser et la superposition de plusieurs impulsions laser permettant d'obtenir la rugosité et/ou la structuration souhaitées.

2. Procédé selon la revendication 1, **caractérisé en ce que** la rugosité et/ou la structuration sont formées au moyen d'un laser à l'état solide, d'un laser excimer ou d'un laser à femtoseconde.

3. Procédé selon les revendications 1 ou 2, **caractérisé en ce que** la rugosité et/ou la structuration sont formées en formant à des distances prédéterminées des découpes d'une nature prédéterminée.

4. Procédé selon l'une des revendications précédentes, **caractérisé en ce que** la découpe présente une lisière dont la rugosité est adaptée au revêtement.

5. Procédé selon au moins l'une des revendications précédentes, **caractérisé en ce que** la structuration formée est en grille, en lignes ou stochastique.

6. Procédé selon au moins l'une des revendications précédentes, **caractérisé en ce que** la rugosité et/ou la structuration sont formées sélectivement dans différentes parties.

7. Procédé selon au moins l'une des revendications précédentes, **caractérisé en ce qu'**on arrondit les chants et les nervures de l'implant à l'aide de l'apport d'énergie électromagnétique.

8. Procédé selon au moins l'une des revendications précédentes, **caractérisé en ce que** la rugosité et/ou la structuration et en particulier certaines découpes qui correspondent à la cicatrisation à laquelle on s'attend sont formées et **en ce que** le traitement de la surface de l'implant est restreint aux parties qui sont dotées avantageusement du revêtement.

9. Procédé selon la revendication 8, **caractérisé en ce que** les découpes sont formées en fonction du volume souhaité pour le revêtement.

10. Procédé selon au moins l'une des revendications précédentes, **caractérisé en ce que** l'on y applique un revêtement actif et/ou un revêtement passif.

11. Procédé selon au moins l'une des revendications précédentes, **caractérisé en ce que** la surface intérieure et/ou la surface extérieure de l'implant sont polies mécaniquement et/ou électrochimiquement.

12. Procédé selon au moins l'une des revendications précédentes, **caractérisé en ce que** la totalité de la surface de l'implant est polie.

13. Procédé selon au moins l'une des revendications précédentes, **caractérisé en ce que** la rugosité et/ou la structuration sont formées au moyen d'un apport d'énergie électromagnétique en impulsions d'une durée inférieure à 1 µs et sont de 0,5 à 5 µm.
